# EUROPEAN PATENT APPLICATION

(11) **EP 4 360 571 A1**
(43) Date of publication of application: **01.05.2024**
(21) Application number: 22204785.4
(22) Date of filing: 31.10.2022
(51) Int. Cl.: A61B 17/22

(54) **SYSTEM AND METHOD FOR REMOVING KIDNEY STONE FRAGMENTS**

(71) Applicant: Wismed Pl Sp Z.o.o. A Company Incorporated in Poland; Number Krs 0000751368, Wroclaw (PL)
(72) Inventor: Wisniewski, Pawel, 31/2 Wielka (PL)
(74) Representative: Straus, Alexander

(57) **Abstract**

A system for guided removal of particulate matter from a subject's body, in particular for removing small stone fragments from the kidney of a subject is provided. The system comprises an ureteric kidney access sheath, a kidney irrigation catheter, an image-forming device and a dual action pumping device. The ureteric kidney access sheath comprises a fluid exhaust port connected to a dual action pumping device. The kidney irrigation catheter comprises a fluid inlet port, connected to the dual action pumping device. Also a method for removing particulate matter from a subject's kidney is disclosed which comprises use of the system.

## Description

### TECHNICAL FIELD

The present invention relates to systems and methods for a guided removal of particulate solid matter, in particular kidney stones from a subject's body.

### BACKGROUND

Urolithiasis is a common disorder involving formation and growth of insoluble deposits in a subject's kidney. These deposits, generally termed kidney stones, are made up from chemicals in the urine resulting in essentially four stone-types based on their constituents: calcium oxalate, uric acid, struvite, and cystine. Common symptoms or urolithiasis include severe pain in the back, urine that smells bad, looks cloudy or even contains blood, nausea, vomiting, fever and chills.

In industrialized countries this disorder affects about 10 % of the population and is mainly based on modern lifestyle including food with too much salt or sugar, obesity and lack of sufficient exercise along with drinking too little of water.

After formation the stone once formed may stay in the kidney not resulting in too much of distress and may even remain undetected in case not causing any troubles. Yet, the stone may also travel down the urinary tract into the ureter and further. Small stones that move out of the body together with the urine do in most cases not cause too much pain. Yet, larger stones may get stuck during their travel and may cause a back-up of urine in the kidney, the ureter, the bladder or the urethra, which may turn out to be extremely painful for the subject affected.

Today a variety of different treatments of urolithiasis is available, being directed either to a straight physical removal of the stones and/or involving a prior size reduction thereof, such as uteroscopy, percutaneous nephrolithomy, nephrolithotripsy or extracorporeal lithotripsy.

In practice the different methods are basically used singly or in combination based on the form and size of the stone or stones to be removed. Percutaneous nephrolithomy, is mainly applied in patients with large or irregularly shaped kidney stones or people with infections, the methods yet requiring to access the kidney through a small incision in the back. The treatment usually necessitates the patient to be anesthetized and to stay hospitalized for about 2 to 3 days.

Among lithotripsy methods shockwave lithotripsy is a widely used treatment process and ensues targeting stones with shock waves from outside the body causing the stone be broken into small pieces. The treatment normally comprises arranging a water filled cushion on the patient's abdomen or behind the kidney and positioning the body such that the stone can be accurately targeted. Depending on the degree of fragmentation the result of the method is "stone fragments" or even "stone dust", pieces assumed to be small enough to pass the urether along with the urine. The main advantage of this kind of therapy is that the patients may be treated by extracorporeal means so that complications due to surgical interventions, duration of hospital stays, costs, recovery periods etc. are greatly reduced.

Recently also laser lithotripsy has attracted a great deal of interest. Instead of using sound waves as the energy source applied from outside the body, laser lithotripsy uses the energy pulse of a laser beam, applied directly to the stone inside the body, e.g. the urether. As such this technique involves directing the laser device directly to the stone prior to firing, e.g. via an endoscope.

Even though lithotripsy is very well accepted among attending physicians due to easy handling, shorter hospitalization etc., there is still a problem in that after "fragmentation" or "dust formation", the particulate matter created, even of small size, is not fully flushed out by regular urine excretion but remains in the kidney cavities or poles, biologically designed to collect urine prior to excretion. In particular, based on gravity, the small fragments or dust pieces mainly remain in calyxes of the lower pole, eventually serving as new seed crystals for the anew formation of larger stones. In fact, according to recent reports, recurrence of the disorder in up to 60% of patients is observed after treatment.

US2017319776 A1 discloses systems and methods for a guided removal of objects from a subject. The method comprises, guiding a flexible tube through a passageway of the subject, wherein the flexible tube comprises a first passageway and a second passageway, positioning a distal end of the first passageway in proximity to the object, infusing liquid through the second passageway and removing the object through the first passageway with at least a portion of the liquid while suction is not being provided.

US2021204968 A1 discloses systems, devices, and methods for the removal of objects from a body. The device may be a urethral catheter configured to aspirate kidney stones from the urinary tract through one or more aspiration ports at the distal face or along a lateral side of the catheter. The catheter may include one or more irrigation ports at the distal face or along the lateral side of the catheter for dislodging kidney stones.

US2021022757 A1 discloses a method for removing a stone from a patient comprising the steps of providing a suction evacuation assembly which includes a sheath and one or more side arms; inserting and positioning a distal end of the sheath into a lumen or cavity of a patient's body containing a stones; connecting a tube to one of the side arms and to a collection bottle; connecting another tube to the collection bottle and a negative pressure system; visualizing the stone or foreign body using a scope inserted through the assembly; activating the negative pressure system in order to remove the stone from the cavity if the diameter of the stone is narrower than an inside diameter of the sheath and the side arm, or performing a lithotripsy on the stone to create fragments with a decreased diameter which allow the passage through the assembly; and collecting the stone in the collection bottle.

Another problem when trying to flush out small objects from a body resides in preventing rise of intrarenal pressure and in keeping an essential constant pressure in the body or organ to be treated, which the prior art devices do not adequately address.

A problem of the present invention thus resides in overcoming the shortcomings of the prior art providing a system and methods for removing essentially the entire particulate kidney stone material from a subject's kidney so as to reduce recurrence of urolithiasis, while at the same time providing a pressure in the kidney to be treated that does not substantially exceed clinically accepted limits.

### SUMMARY OF THE INVENTION

The present invention solves the above problem by providing a system and a method for removal of small fragments from the kidney of a subject. The system comprises an ureteric kidney access sheath, a kidney irrigation catheter, an image-forming device and a dual action pumping device. The ureteric kidney access sheath comprises a fluid exhaust port connected to a dual action pumping device. The kidney irrigation catheter comprises a fluid inlet port, connected to the dual action pumping device.

The ureteric kidney access sheath is designed and constructed to receive the kidney irrigation catheter therein and to have a form and volume to allow fluid and small pieces to pass between the inner wall of the ureteric kidney access sheath and the outer wall of the kidney irrigation catheter. The image-forming device is configured to provide a picture of the kidney and or its poles and calyxes , in particular an area before and around the irrigation catheter's tip and to assist in guiding the same during operation. The image-forming device may be a camera arranged at the tip of the irrigation catheter and comprising a light source. Alternatively, an ultrasonic device may serve as such imaging device. For better visualization of the kidney's areas the image forming device may be connected with a display device.

The system further comprises a dual action pumping device allowing an essentially concomitant pumping and draining activity. The fluid outlet port of the ureteric kidney access sheath is connected with a side of the dual action pumping device providing suction while the fluid inlet of the kidney irrigation catheter is connected with another side of the dual action pumping device providing a build-up of pressure.

The invention further provides a method for removing small kidney stones, fragments and/or stone dust from a subject's kidney, as e.g. obtainable after a lithotripsy procedure, which comprises the use of the above system on and in an individual to be treated. The method comprises providing the system of the present invention, inserting the ureteric kidney access sheath containing the kidney irrigation catheter therein into a subject's kidney, swirling the small pieces, stone fragments and/or stone dust present in the kidney and/or its poles by effecting an influx of fluid via the dual action pumping device through the kidney irrigation catheter into the kidney and/or its poles and calyxes and essentially at the same time providing an efflux of fluid present in the kidney and/or its poles and calyxes containing dispersed material via the dual action pumping device through the lumen provided in the kidney access sheath, and repeating the operation one or more times with the tip of the irrigation catheter being turned or displaced relative to its antecedent position.

### BRIEF DESCRIPTION OF THE DRAWINGS

The invention will now be described by the Figures in which:
FIG. 1 shows a schematic scheme of a preferred embodiment of a system for removing kidney stones according to the present invention;
FIG. 2A shows an artificial kidney model filled with saline and kidney stones.
Fig 2b shows the artificial kidney model after treatment with the system of the present invention.

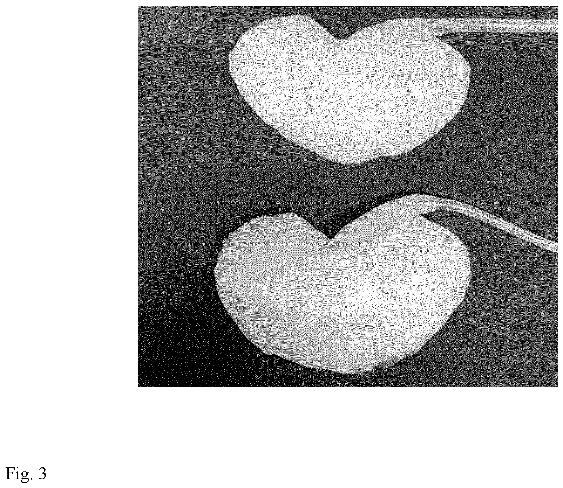

Fig. 3 shows a kidney model exhibiting the anatomical shape, dimension and flexibility of a biologic kidney.

### DETAILED DESCRIPTION OF THE INVENTION

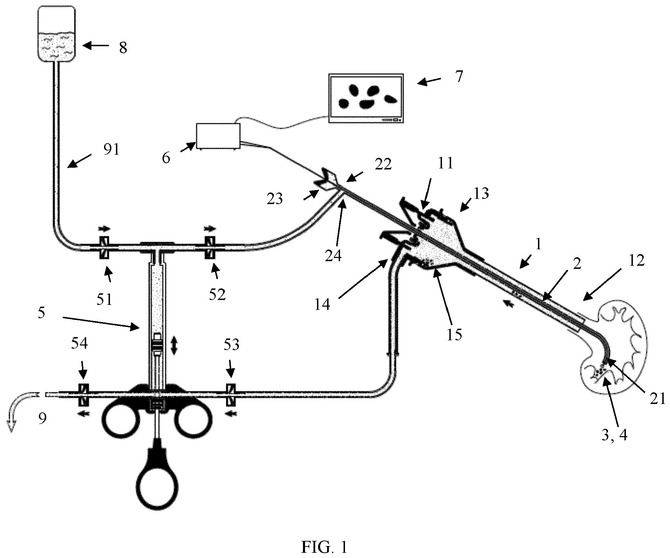

The invention will now be described with reference to Fig. 1 showing a preferred embodiment of the system of the present invention.

The system of the present invention comprises a ureteric kidney access sheath 1, a kidney irrigation catheter 2, an image-forming device 3 and a dual action pumping device 5.

The ureteric kidney access sheath 1 is a flexible hollow tube and conventionally used in the art for providing access to a kidney. It is made of a material acceptable for application in humans. The ureteric kidney access sheath 1 to be used in the resent invention has a form and volume allowing insertion thereof through the urinary bladder and ureter up to the kidney and is further designed to receive the kidney irrigation catheter 2 via an access port 11, while leaving sufficient space and volume between the inner wall of the ureteric kidney access sheath 1 and the outer wall of the kidney irrigation catheter 2 to allow fluid and small solid particles to pass and being transported with a fluid flow. This may be embodied such that an inner lumen of the ureteric kidney access sheath has a larger diameter than an outer circumference of the kidney irrigation catheter to be used. As a consequence, the kidney irrigation catheter does not occupy the entire space of the ureteric kidney access sheath and fluid may pass within the sheath besides the catheter. Ureteric kidney access sheath

According to an embodiment the ureteric kidney access sheath 1 may comprise spacers along its length arranged at the circumference of the inner wall and designed to maintain the kidney irrigation catheter 2 arranged in the ureteric kidney access sheath 1 essentially in the middle of its lumen. These spacers may be arranged along the length of the ureteric kidney access sheath 1 or essentially only at one or both ends 12, 13 thereof. According to a preferred embodiment one or more spacers are provides at the end 13 of the ureteric kidney access sheath 1 so as to keep kidney irrigation catheter 2 in a central position, which improves sealing functionality.

The ureteric kidney access sheath 1 has a distal end 12 to be inserted into the kidney's lumen, and a proximal end 13, where an outlet port 14 for fluid to be discharged and an access port 11 for inserting the kidney irrigation catheter 2 is provided.

According to an embodiment the proximal end 13 of the ureteric kidney access sheath 1 has a bigger size or diameter, i.e. volume, than the ureteric kidney access sheath 1 as such, allowing an improved discharge of the fluid to be drained and an easier access for the kidney irrigation catheter 2. Such enlarged volume also serves as a primary sedimentation chamber for particulate material 15. Such increased volume may be provided integrally in the ureteric kidney access sheath 1 as such or as an additional drainage chamber connected with the ureteric kidney access sheath 1, which chamber comprises a coupling passage for connection with the ureteric kidney access sheath 1 in the form of a mouth defined at the opened up end of the chamber and an instrument insertion aperture in alignment with the mouth and opposite to the coupling passage in the chamber, which may be formed with penetrable sealing means and defining a central location of instrument penetration along which the kidney irrigation catheter 2 which is sealably insertable.

Also, the ureteric kidney access sheath 1 or at least the proximal part 13 thereof, in particular the sedimentation chamber 15, is made of a transparent material allowing a visual inspection from the outside, e.g. determining and checking, whether some, or after rounds of treatment cycles, still some particulate matter 15 accumulates, which assist the attending physician in determining, whether or not any particulate matter is still present in the kidney and may be drained therefrom, even though not visible via the image-forming device 3 anymore.

In principle, the image forming device may be any device or apparatus capable to provide a picture, e.g. presented via a monitor, to the attending personal of the spatial environment the kidney irrigation catheter 2 is moving in or around. Examples for such image forming devices are cameras arranged at the tip of the kidney irrigation catheter 2, or ultrasonic devices, which may be arranged outside the subject or at the tip of the kidney irrigation catheter 2 as well. It also may be an autonomous insertable small bore endoscope camera .

In case of using a camera, in particular a micro-camera, arranged at the tip of the kidney irrigation catheter 2 also a light source 4 may be provided to enable visualisation of the environment in front of and/or around the kidney irrigation catheter 2. The image forming device arranged at the tip of the kidney irrigation catheter 2 may be connected to control and/or display means via a wire or wireless, with wire connections being sized, adapted and arranged in the ureteric kidney access sheath 1 or at the kidney irrigation catheter 2, so as not to interfere with efflux fluid containing particulate matter to be drained.

The image forming device assists the attending personal during operation to guide the kidney irrigation catheter 2 to a treatment area, e.g. into a predetermined kidney's pole, and/or to visualize the content thereof, i.e. whether some or some remaining particulate matter may be detected.

The fluid outlet port 14 is sealingly connected with a tube or pipe 9, adapted to be connected with the dual action pumping device 5 and eventually a container for collecting drained fluid (not shown).

During operation the fluid outlet port 14 of the ureteric kidney access sheath 1 may be arranged such that the outlet 14 is below the inlet or opening 11 for the kidney irrigation catheter 2, which assists in removing fluid inclusive kidney stone fragments. Also, when the outlet of the tube 9 is arranged in height below the individual's kidney the ureteric kidney access sheath 1 may provide a syphoning functionality. In order to safeguard such syphoning functionality during operation, during which the tube 9 may be moved to some extent by the attending personnel, the ureteric kidney access sheath 1 may provide an elongated outlet tubing 14' manufactured integrally with the kidney access sheath 1 or attached to the fluid outlet port 14. This outlet tubing is preferably made of a rigid material, so that it may be directed during operation always downwards. In case of using tubing 14', the tube 9 then connects with the outlet of the tubing 14' and is in turn connected dual action pumping device 5.

The access port 11 in the ureteric kidney access sheath 1 for inserting the kidney irrigation catheter 2 may be sealed by any conventional means e.g. with an O-ring made of a material usually used in surgical procedures and designed to tightly and sealingly enclose the kidney irrigation catheter 2 while at the same time serving as a sealing plug for the kidney irrigation catheter 2 inlet port.

Pipe or conduit 9, which is preferably prepared of a flexible, transparent material, is fluidly connected to a dual action pumping device 5, which at the point of connection with pipe 9 provides for a suction and draining activity, i.e. a fluid flow away from the outlet port 14 only. This may be achieved by any means known in the art, e.g. providing a vacuum pump and/or also by arranging a one-way valve 53 in the pipe 9 between the fluid outlet port of the ureteric kidney access sheath 1, configured to let fluid flow from the outlet port in the ureteric kidney access sheath 1 to the pumping device only. According to a preferred embodiment a second one-way valve 54 may be provided in pipe 9 downstream of the dual action pumping device 5 for avoiding sucking up fluid into the dual action pumping device 5 already transported downstream.

The system further comprises a kidney irrigation catheter 2 adapted to be inserted into the ureteric kidney access sheath 1 via access port 11. The kidney irrigation catheter 2 is in principle a flexible pipe comprising a distal end 21 and a proximal end 22 and with a lumen suitable for transporting fluid. The kidney irrigation catheter 2 further comprises means 23 conventionally used in the art for guiding the distal end of a catheter to a desired location, e.g. by flexing or turning or forward motion to a kidney's cavity/pole to be rinsed/cleaned, and if provided also may comprise means for connecting with a camera 3 and a light source 4 arranged at the distal end 21, such as e.g. wires, Bluetooth devices etc..

The proximal end 22 of the kidney irrigation catheter 2 comprises a fluid inlet port 24 fluidly connected to the dual action pumping device 5 at a connection point via pipe or conduit 91, where fluid may be pumped in at a predetermined pressure suitable for biological conditions. The fluid inlet port 24 may preferably be arranged at and mounted to the kidney irrigation catheter 2 in a manner not obstructing a 360° turn of the catheter 2, while constantly providing a seal. The dual action pumping device 5 and/or the connection at this point is designed to allow a pumping activity only, which may be achieved by any means known in the art, e.g. by providing a pressure pump and/or by means of a one-way valve 52 arranged between the dual action pumping device and the fluid inlet port in the kidney irrigation catheter 2. The pipe 91 may be of a flexible and transparent material and may preferably comprise a section prior to the fluid inlet port 24, that has a higher degree of flexibility as the remaining part of pipe 91, such providing a damping or buffering effect of any pressure applied by the dual action pumping device 5. The dual action pumping device 5 is connected with a fluid reservoir 8, preferably via a one-way valve 51, adapted to direct any pressure to be exerted in the direction of the kidney irrigation catheter 2 only.

The fluid reservoir 8 contains the fluid for the treatment, which may be any fluid usually used in medicine for rinsing organs, and is physiologically acceptable, e.g. pharmaceutical grade saline.

In operation the ureteric kidney access sheath 1 is introduced into the subject via the urethra, the urinary bladder and through ureter up to the kidney of a subject upon which the kidney irrigation catheter 2 is inserted into the ureteric kidney access sheath 1. Introduction of the ureteric kidney access sheath 1 into the kidney is advanced up to the pelvis of the kidney, which ensures that there is sufficient space for the kidney irrigation catheter 2 to protrude out of the distal end 12 of the ureteric kidney access sheath 1 and to be guided into the different kidney poles to be flushed. Likewise, the ureteric kidney access sheath 1 may be introduced into a subject with the kidney irrigation catheter 2 already be installed therein.

The kidney irrigation catheter 2 protrudes out of the ureteric kidney access sheath 1 such that it may conveniently be directed, e.g. bent, rotated, turned and/or displaced into the different kidney poles and calyxes by the attending personnel via means 23. The distal end 12 of the ureteric kidney access sheath 1 may be positioned at the pelvis of the kidney, but may likewise also be advanced somewhat more into the kidney's lumen and more adjacent to a respective kidney's pole, where the treatment is ongoing.

The volume of fluid to be pumped into the kidney will be adjusted by the skilled person based on subject's parameters, e.g. age and the like, but also by the position of the ureteric kidney access sheath 1 when flushing a pole. Specifically, when arranging the ureteric kidney access sheath 1 closer to a pole to be flushed, the volume of fluid to be used will be selected smaller, as compared to positioning the ureteric kidney access sheath 1 at the pelvis, so as to observe an equilibrated pressure in the subject's kidney. Likewise, in this adjacent positioning the pump and drain activity may preferably be selected to be effected at the same time.

In principle, for a flush a volume of from about 0.5 - 2.5 ml is used, preferably from about 0.75 ml to about 2 ml, more preferably from about 1 ml to about 1.5 ml. By following an essentially concomitant flush and drain activity the pressure created inside the kidney may well be maintained below the clinically accepted level of about 40 mm Hg. According to the present invention during operation, the pressure level is held below about 35 mm Hg, more preferably below about 30 mm Hg, even more preferred below about 25 mm Hg, and even more preferred below about 20 mm Hg.

In order to fully flush and drain a kidney's calyxes in all poles the pumping/draining action is repeated several times in a pole, with the kidney irrigation catheter 2 being angled or turned for each flush in a degree of e.g. 10°, 20°, 30°, 40°, 50°, 60°, 70°, 80°, 90°, 100°, 110°,120°, 160°, 180° or 240 ° so that the kidney irrigation catheter 2 turns at least once by 360° degree in the pole in order reach all areas in the poles and to disperse all of the particular matter present therein. For the ease of performance, the turn may be to the right or to the left only, but likewise a combination of turning to the right and to the left in different angles is feasible, e.g. 30° to the right and subsequent 60° to the left or vice versa.

In an alternative embodiment the system or the kidney irrigation catheter 2 and/or the ureteric kidney access sheath 1 may further comprise a pressure sensor designed to sense the kidney's internal pressure assisting an attending personnel to adjust the volumes and/or flow rate and/or pressure of the fluid influx and efflux so that the limits of physiological pressures normally prevailing in a kidney are not exceeded.

Preferably, the system further comprises a processing unit 6 and a monitor 7 adapted to compute, display and/or store pictures send from the camera. Likewise, the light source 4 may also be operated via the processing unit 6 or separately.

After initial kidney stone fragmentation and evaluation of the prevailing situation of the subject to be treated, e.g. scrutinizing her or his anamnesis and/or pre-treatment history, the attending personnel will position the ureteric kidney access sheath 1 and the kidney irrigation catheter 2 in the subject as detailed above and activate the dual action pumping device 5, which is adapted to provide a fluid inflow into and essentially simultaneously a fluid outflow out of the kidney, such that the pressures, a kidney is exposed under normal biological conditions is essentially not exceeded.

In principle the dual action pump device 5 may be embodied by separate pressure and vacuum pumps providing and effecting a fluid influx via the kidney irrigation catheter 2 and a fluid efflux via the ureteric kidney access sheath 1, respectively. Both pumps are connected and adapted to meet the requirements of balanced pressure conditions in the kidney, that is the pressure pump provide a volume of fluid, while the vacuum pump drains the same volume, both activities being performed at essentially at the same time. According to an embodiment the draining activity may be delayed to some extent to be able to drain fluid that contains as much dispersed material as possible.

Alternatively, the dual action pump device 5 may also be embodied as an entity of a one stroke dual action piston pump, as exemplified in Fig. 1, having a pumping piston within a cylinder. One pumping stroke of the piston is used to simultaneously provide pumping pressure and suction force for fluid inflow and fluid suction, respectively at opposite sides of the piston.

The dual action pumping device 5 may be an isovolumetric pumping/suction device and may be adapted for hand operation or for foot operation.

The dual action pumping device 5 pumps fluid into a kidney's cavity via the kidney irrigation catheter 2 with a volume and rate sufficient to disperse particulate matter present in the fluid. Essentially at the same time the draining activity of the dual action pumping device 5 is initiated, so that any excess fluid pumped into the kidney, and now containing the dispersed particulate matter, is actively drained (aspirated ) via the ureteric kidney access sheath 1.

According to a preferred embodiment, the fluid volume pumped into the kidney, is essentially equal to the volume of fluid sucked out of the kidney. Preferably, the same volume of fluid is simultaneously pumped into the kidney per time unit, to what is sucked out of the kidney, such that the fluid volume within the kidney is essentially kept constant.

According to an embodiment the fluid is pumped into the kidney in pulsed small volumes, while the draining action of the pump 5 is initiated either simultaneously or only after e.g. 2 or 3 or 4 pulsed volumes and designed to drain the entire volume as pumped in.

The pumping/draining activity may be automatically controlled e.g. via a software run on a computer or linked to a manually activated pumping operation. The procedure is such that essentially not more fluid is drained as compared to what has been pumped in and vice versa, while always considering the pressure limits in the kidney.

The operation is repeated in the respective pole started with while turning or displacing the tip of the catheter 22 in an angular or advancing or retracting manner until no particulate matter may be observed with the camera or in the sedimentation chamber. Then the next pole is treated.

Alternatively, the flushing may also be performed continuously, wherein in in case of a syphon functionality the hydrostatic pressure of continuously flowing liquid is adjusted by changing the altitude of bag 8, while the outflow may be adjusted by adjusting the altitude of pipe 9. The pumped volumes (bolus volume) as opposed to volumes from hydrostatic pressure irrigation flow are in range maximum up to 2,5 ml. These volumes of irrigation are coupled with the same volumes being aspirated from the kidney just forming isovolumetric system of irrigation-suctioning.

In summary, the present system provides a fluid flow into the kidney via the distal part 21 of the kidney irrigation catheter 2, while providing a draining flow from the outlet port 14 in the proximal part 13 of the ureteric kidney access sheath 1 of the same pressure/volume. Thus, the intrarenal pressure remains essentially constant. The ureteric kidney access sheath 1 may be guided in any direction in the kidney's lumen to provide a fluid outflow from the kidney pelvis to be flushed in order to entirely remove particulate matter, such as kidney stone fragments.

The invention further provides a method for removing particulate matter, i.e. small kidney stones or fragments and stone dust, from a subject's kidney, e.g. as obtained after a lithotripsy procedure, which comprises use of the above system. The method comprises the steps of providing the system of the present invention, inserting the ureteric kidney access sheath 1 containing the kidney irrigation catheter 2 therein into the lumen of a subject's kidney, guiding the kidney irrigation catheter 2 to a kidney's pole and individual calyxes respectively, and introducing a volume of fluid from a reservoir 8 via a dual action pumping device 5 and pipe 91 into the kidney irrigation catheter 2 and into the kidney's pole, thereby swirling up particulate matter contained in the pole and its calyxes At the same time or in case shortly afterwards the fluid containing particulate matter dispersed by the flushing action is drained either from the pole or from the kidney's lumen and the flush/draining operation is repeated.

The anew flush/drain operation is preferably carried out after displacing the distal end 21 of the kidney irrigation catheter 2, e.g. after turning the distal end 21 by a predetermined degree.

In order to safeguard the normal pressure limits of a kidney the attending personel may first check the existing volume of the kidney to be treated and optionally fill the kidney prior to starting the treatment procedure. In an alternative embodiment, once a full volume has been determined, e.g. by determining the inside pressure of the kidney to be close to the upper pressure limit, a volume of fluid may be drained via the ureteric kidney access sheath 1 before the pumping/draining operation is initiated, so that any, even small pressure deviations due to the pumping/draining operation may be kept within physiological levels.

The invention has been described above with reference to the Figures and particular embodiments. It is to be noted that features described in connection with a particular embodiment are expressible combinable with other embodiments or features thereof as long as they do not technically exclude each other.

The following examples shall illustrate the invention without limiting the same thereto.

### Example 1: Depletion of Solid Particles

The operational efficacy of the system has been tested utilizing an artificial kidney model, reflecting characteristics of the human kidney regarding the shape of the interior kidney cavities/ poles/pockets. The validated kidney models (https://link.springer.com/article/10.1007/s10439-016-1757-5) have been obtained from Max Planck Institute for Intelligent Systems and Institute of Physical Chemistry, University of Stuttgart, Germany. They are made of silicon exhibiting a similar modul of elasticity as human kidneys and are translucent to be able to observe any internal procedures from the exterior. The models exhibit two openings at opposite ends along its length axis one lower opening being designed to reflect the size and diameter of a renal pelvis, the other upper opening having a diameter to receive a pressure sensor. Further, the models comprise pockets, resembling kidney's poles/calyxes in form and shape. For determining the pressure, the Fiber Optic Pressure sensor FOP-M200, supplied by FISO Technologies Inc., Quebec, Canada has been used.

The pressure sensor is inserted into the respective opening of the model, which is sealed by Tuohy -Borst sealing connector, obtained from Qosina Corporation , USA. Then 0.33 g human kidney stones obtained from uteroscopy procedures from patients, crushed, and sorted by sieving for stones/fragments having an average size of about 1 mm were inserted in the model through the lower opening.

In a next step an ureteric access sheath (UAS), obtained from Wismed PL Sp. Z o.o., Wroclaw , Poland was inserted into the lower opening of the model establishing a sealingly closure between the outer wall of the UAS and the inner wall of the lower opening by means of tight fit and compression of the artificial ureter.

The model was then fully filled with 8 ml saline (0.9 % NaCl) via the lumen of the UAS at a temperature of about 37.5°C, reflecting body temperature.

A dual action pump obtained from Wismed PL Sp. Z o.o., Wroclaw , Poland was connected with its suctioning arm to the proximal end of the UAS via a flexible, translucent pipe and to source of irrigation and steerable catheter with its pumping arm. The dual action pump contains 4 one-way valves arranged as shown in Fig. 1.

A catheter obtained from Wismed PL Sp. Z o.o., Wroclaw, Poland and connected via pipes with the dual action pump was inserted into the UAS such as visibly protruding into the model and the model was shortly agitated to distribute the stones in the different pockets (calyxes) of the model and laterally placed on an aluminium bowl, allowing the stones to sediment and to stay in the respective pockets.

The reservoir bag, containing the saline was arranged about 110 cm above the kidney model and the outlet for the pipe 9 draining saline (0.9 % NaCl) was arranged about 30 cm below the kidney model.

The reservoir bag was opened to supply saline (0.9 % NaCl) via the pipes to the model. The basic pressure inside the model was determined 10 x over 5 min to be 9.97 mm Hg mean.

The catheter was then advanced to a pocket and the dual action pump was initiated by hand providing a flush with a total volume of about 2,5 ml / 1 Sek, injected into the pocket upon a stroke.

After a few strokes the tip of the catheter was turned in the same pocket by and the stroke/flushing was repeated. In this example each pocket of the model was treated with 5 number of flushes and entire interior of the kidney has been flushed. A clear swirling of the stones in the pocket was observed upon each flush, which was even pronounced after angularly displacing the tip. A general distribution of the stones in the entire liquid lumen of the model was noted after 6 strokes /flushes in the first pocket, which progressed after further strokes, but diminished after 10 number of strokes/flushes due to the stone displacement towards tip of Ureteric kidney access sheath and further arriving at the UAS being drawn off.

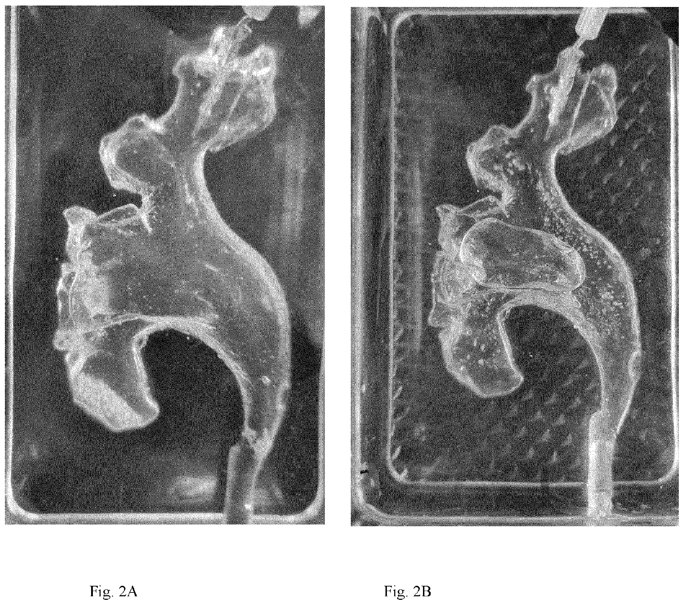

After repeating the strokes/flushes as above in the other pockets of the model a final series of 4 strokes/flushes was effected in the lumen of the model with the result that essentially all stones could be drained therefrom. The entire procedure required about 5 minutes resulting in about 92% of the initial stone fragments were to be removed as determined by dry weight from the initial amount introduced and the amount remaining. The result is shown in Fig. 2B.

When repeating the procedure after an initial run the removal efficacy could be increased to about 96 %.

The pressure measured during the procedure remained below 30 mm Hg which is lower than the 40 mmHg threshold level considered to be safe.

### Example 2: Pressure Maintenance

In order to show the capability of the system to establish and keep a pressure that is within acceptable safe range, the kidney model of example 1 was used, which reflects spatial and mechanical physiological characteristics of a kidney.

In this experiment the same set up as in example 1 for the catheter, the UAS and the dual action pump was used, with the proviso that the pressure sensor (Fiso, supra) was inserted into the model's lumen via the UAS. In addition, the catheter contained a camera at its tip to ensure proper arrangement of the catheter in a pocket.

The reservoir bag and the outlet were arranged at essentially the same level as the kidney model. The saline reservoir was opened to provide a constant flow of saline into the kidney model. The initial pressure was determined 7.84 mm Hg mean, which is within the normal intrarenal pressure of 0 - 10 mmHg.

The dual action pump was activated by repeatedly injecting flushes of about 2,5 ml into a pocket. The tip of the catheter was turned in each pocket once before going to another pocket-

The mean pressure variation during the pump and drain operations could be determined to be within a range of between about 20-30 mm Hg, which is well below the 40 mm Hg pressure considered to be safe.

## Claims

1. A system for removal particulate matter from the kidney of a subject, comprising:
a ureteric kidney access sheath (1);
a kidney irrigation catheter (2), configured to be inserted into the kidney access sheath (1) and adapted to provide a lumen between the inner wall of ureteric kidney access sheath 1 and the outer wall of kidney irrigation catheter 2 sufficient to allow a fluid flow containing particulate matter therethrough;
a picture-forming device (3) configured to provide a picture of the treatment area; and
a dual action pumping device (5), providing an essential simultaneous fluid influx into the kidney irrigation catheter 2 and a fluid efflux in essentially the same volume in the ureteric kidney access sheath 1.

2. The system of claim 1, further comprising a sedimentation chamber (15) for collecting the removed particulate matter, wherein the sedimentation chamber (15) is fluidly connected to the ureteric kidney access sheath (1) and to the dual action pumping device (5).

3. The system of claim 2, wherein the sedimentation chamber (15) allows visual observation of the collected particulate matter.

4. The system of claim 1, wherein the kidney irrigation catheter (2) comprises a picture forming device at the tip thereof.

5. The system of any of the preceding claims, wherein the picture-forming device (3) is a camera or an ultrasound device.

6. The system of any of the preceding claims, wherein the dual action pumping device (5) is a dual action piston pump, wherein one pumping stroke of a piston of the dual action piston pump is used to simultaneously provide pumping pressure and suction force for fluid inflow and fluid efflux, respectively.

7. The system of any of the preceding claims, wherein the pumping device (5) is an isovolumetric pumping/suction device.

8. The system of any of the preceding claims, wherein the dual action pumping device (5) is adapted for hand operation or for foot operation.

9. The system of any of the preceding claims, the system further comprising:
a first one-way valve (51), which is positioned between a fluid connection of the dual action pumping device (5) and a fluid supply (8), configured to only let fluid flow from the supply (8) to the dual action pumping device (5);
a second one-way valve (52), which is positioned between the pumping device (5) and the inner channel (22) of the steerable kidney irrigation catheter (2), configured to only let fluid flow from the pumping device (5) to the inner channel (22);
a third one-way valve (53), which is positioned between a fluid connection of the outlet port (14) and the dual action pumping device (5), configured to only let fluid flow from the outlet port (14) to the pumping device (5); and
a fourth one-way valve (54), which is positioned between a fluid connection of the dual action pumping device (5) and a waste fluid outlet (9) of the system, configured to only let fluid flow from the dual action pumping device to the waste fluid outlet (9).

10. The system of any preceding claims, the system further comprises a processing unit (6), wherein the image forming device (3) is connected to the processing unit (6) and the processing unit (6) is connected to a monitor (7), to enable the operator to observe and store visual data, obtained by the image forming device (3).

11. A method for removing particulate matter from a subject's kidney comprising:
providing a system according to any of the preceding claims; and
repeatedly effecting a pumping/draining operation; and
collecting the particulate matter from the subject's kidney.

12. The method of claim 9 or the system according to any of the claims 1-8, wherein an intrarenal pressure is not increased during operation of the system above 40 mm Hg.

13. The method of claim 9, wherein the method is performed after the subject has been subjected to a lithotripsy.

14. The method of any of claims 9 - 12 or the system according to any of the claims 1-8,
when the shape of the fragments is substantially irregular, the largest extension of a small fragment is in the range of 200-2500 µm,
preferably the largest extension does not exceed 1 mm or the fragment extension is substantially 1 mm in average; or
when the shape of the fragments is substantially round, the diameter of the fragments is in the range of 200-2500 µm, preferably the diameter does not exceed 1 mm or the fragment diameter is substantially 1 mm in average.

15. The system of any preceding claim, wherein the small fragments have been produced by fragmenting or by dusting.
